# EUROPEAN PATENT SPECIFICATION

(11) **EP 1 603 550 B1**
(45) Date of publication and mention of the grant of the patent: **24.12.2008**
(21) Application number: 04719398.2
(22) Date of filing: 11.03.2004
(51) Int. Cl.: A61K 31/195, A61K 9/08

(54) **PHARMACEUTICAL COMPOSITION COMPRISING 5-METHYL-2-2'-(CHLORO-6'-FLUOROANILINO)PHENYLACETIC ACID**
PHARMAZEUTISCHE ZUSAMMENSETZUNG ENTHALTEND 5-METHYL-2-2'-(CHLORO-6'-FLUOROANILINO)PHENYLESSIGSÄURE
COMPOSITION PHARMACEUTIQUE CONTENANT DE L'ACIDE 5-METHYL-2-2'-(CHLORO-6'-FLUOROANILINO)PHENYLACETIQUE

(30) Priority: 12.03.2003 US 454145 P
(43) Date of publication of application: 14.12.2005
(73) Proprietor: NOVARTIS AG, 4056 Basel (CH); NOVARTIS-PHARMA GMBH, 1230 Vienna (AT)
(72) Inventor: FORENZO, Patrick, Glen Gardner, NJ 08826 (US); KHALED, Maha, Y., 4059 Basel (CH); WANG, Barbara, Berkeley Heights, NJ 07922 (US); ZIELINSKI, Joseph, Lawrence, Florham Park, NJ 07932 (US)
(74) Representative: de Weerd, Petrus G.W.
(86) International application number: PCT/EP2004/002528
(87) International publication number: WO 2004/080451

(56) References cited:
- US-A1- 2002 061 932
- US-B1- 6 291 523

## Description

This invention relates to compositions for the treatment of cyclooxygenase-2 mediated disorders and conditions comprising 5-methyl-2-(2'-chloro-6'-fluoroanilino)phenylacetic acid or a pharmaceutically acceptable salt thereof suitable for oral administration, and methods of treatment of cyclooxygenase-2 mediated disorders and conditions by the oral administration of 5-methyl-2-(2'-chloro-6'-fluoroanilino)phenylacetic acid.

All patents, patent applications, and other publications referred to herein are hereby expressly incorporated by reference in their entirety. In case of a conflict between the present specification and material incorporated by reference, the present specification is controlling.

The present invention is directed to a composition for the treatment of cyclooxygenase-2 mediated disorders and conditions, the composition comprising a suspension of 5-methyl-2-(2'-chloro-6'-fluoroanilino)phenylacetic acid. The utility of this compound and methods for its synthesis are disclosed in U.S. Patent 6,291,523.

The present invention is also directed to methods for treating a cyclooxygenase-2 dependent disorder or condition comprising administering an effective amount of the compositions of the invention, i.e., a liquid oral dosage formulation comprising of 5-methyl-2-(2'-chloro-6'-fluoroanilino)phenylacetic acid.

As discussed in U.S. Patent 6,291,523, a genus of compounds, including 5-methyl-2-(2'-chloro-6'-fluoroanilino)phenylacetic acid, is useful for the relief of pain, fever and inflammation of a variety of conditions including rheumatic fever, symptoms associated with influenza or other viral infections, common cold, low back and neck pain, dysmenorrhea, headache, including migraine headache, toothache, sprains and strains, myositis, neuralgia, synovitis, arthritis, including osteoarthritis and rheumatoid arthritis, degenerative joint diseases, gout and ankylosing spondylitis, bursitis, burns, and injuries following surgical and dental procedures. U.S. Patent Application US2002/061932A1 discloses different forms of tablet formulations suitable for oral use, comprising 5-methyl-2-(2'-chloro-6'-fluoroanilino)phenylacetic acid. Some individuals, especially children, have difficulty swallowing solid oral dosage formulations. Thus, it is desirable to provide liquid oral dosage formulations comprising 5-methyl-2-(2'-chloro-6'-fluoroanilino)phenylacetic acid for the treatment of the aforementioned conditions in individuals who have difficulty swallowing solid oral dosage formulations.

It has now surprisingly been found that a shelf-stable liquid oral dosage formulation comprising 5-methyl-2-(2'-chloro-6'-fluoroanilino)phenylacetic acid can be prepared. The 5-methyl-2-(2'-chloro-6'-fluoroanilino)phenylacetic acid drug substance is relatively water insoluble and also degrades in water, and so the ability to produce a shelf-stable formulation was unexpected. Further, it was surprisingly discovered that the suspendability of the 5-methyl-2-(2'-chloro-6'-fluoroanilino) phenylacetic acid drug substance can be highly dependent on the order of addition of the suspension components, in particular the suspending agent and the buffer.

The liquid oral dosage formulations comprising 5-methyl-2-(2'-chloro-6'-fluoroanilino)phenylacetic acid are preferably suspensions of 5-methyl-2-(2'-chloro-6'-fluoroanilino)phenylacetic acid. Suitable suspending agents include microcrystalline cellulose, carboxymethylcellulose sodium, guar gum, xanthan gum, gellan gum, carrageenan, sodium starch glycolate, and mixtures thereof. Concentrations of suspending agent in the formulations of the invention can range between about 0.1% to about 3%, or between about 0.5% and about 2.5%, or between about 1% and about 2%, or about 1.5%.

The formulations of the invention can also contain a wetting agent, e.g., polysorbate 80, poloxamers, including poloxamer 188, polyethoxylated castor oil and polyethoxylated hydrogenated castor oil, and polyoxyl 40 stearate. Poloxamer 188 has the structure HO(CH₂CH₂O)ₐ(CH(CH₃)CH₂OH)_{b}(CH₂CH₂O)_{c}H, where a is 75, b is 30, and c is 75, with an average molecular weight of about 8350. The wetting agent is present in amounts typically between about 0.1% and about 5%, or between about 0.18% and about 1%, or between about 0.18 and about 0.25%, or between about 0.18 and about 0.22%, or about 0.2%.

The pH of the formulation can range between about 4.3 and 5.5, preferably between about 4.5 and about 5.5 or between about 4.75 and about 5.25. The pH can also range between about 4.9 and about 5.0. Suitable buffers include, e.g., alkaline metal citrate buffers, such as alkaline metal citrate salts with citric acid, alkaline metal acetate buffers, such as sodium acetate salts with acetic acid, and alkaline metal succinate buffers, such as sodium succinate salts with succinic acid, and mixtures thereof.

The formulations typically contain an antifoaming agent, e.g., simethicone, typically added as an emulsion, e.g., a 30% emulsion. Such a 30% emulsion can be added at a concentration of about 0.1% to about 0.25% in the final formulation. Sweeteners such as saccharin, sodium saccharin, aspartame, sucralose, acesulfame potassium, glucose, fructose, lactitol, maltitol, maltose, sorbitol, sucrose, and xylitol can be used. Flavoring agents can also be added to improve compliance.

Suitable preservatives for oral suspensions are known to those of skill in the art and include, e.g., benzoic acid, sorbic acid, parabens (butyl, ethyl, methyl, propyl), sodium benzoate, and sodium propionate. A preservative such as those set forth above, or a mixture thereof, can be present in amounts between about 0.01% and about 0.3%; or between about 0.02% and 0.25%; or between about 0.1% and about 0.2%. In one embodiment, the formulation comprises about 0.02% propyl paraben and about 0.18% methyl paraben. Other embodiments include formulations comprising 0.03% propyl paraben and 0.12% methyl paraben, 0.148% methylparaben and 0.016% propylparaben and formulations comprising 0.1 % methyl paraben and 0.1 % sorbic acid.

The suspensions of the invention can be made in conventional liquid formulation equipment. In one embodiment, the suspension of the invention is produced by a process comprising admixing water, drug substance, and suspending agent, followed by the addition and admixture of buffer components. Alternatively the suspension of the invention may be prepared by admixing water, suspending agent and buffer system components, followed by the addition and admixture of the drug substance. It has surprisingly been discovered that a suspension cannot be achieved if the buffer components are admixed with drug substance prior to the addition of suspending agent, when the suspending agent is a mixture of microcrystalline cellulose and sodium carboxymethylcellulose.

A pH of between about 4.3 and 5.5 provides a suspension with the most stable drug substance. Formulations with a pH below 4.3 have increased level of a cyclic degradation product, while those above pH 5.5 have increased levels of an oxidative degradation product. Further, increasing the pH of suspension formulations of 5-methyl-2-(2'-chloro-6'-fluoroanilino)phenylacetic acid above about pH 5.5 results in an undesirable increased solubilization of the drug substance.

### Examples

### Example 1: Formulation

**Table 1**

| Ingredient | Amount (mg/ml) |
|---|---|
| 5-methyl-2-(2'-chloro-6'-fluoroanilino) phenylacetic acid | 10.0 |
| Propylparaben | 1.0 |
| Poloxamer 188 | 2.00 |
| Sorbic Acid | 1.0 |
| Simethicone emulsion 30% | 1.00 |
| Flavor | 4.0 |
| Suspending agent: Avicel® RC591 | 18.00 |
| Propylene glycol | 25.00 |
| Sorbitol solution 70% | 200.00 |
| Citric acid anhydrous | 0.71 |
| Sodium citrate dehydrate | 1.88 |
| Sodium saccharin | 0.50 |
| Water purified, USP | q.s. to 1 ml |

Poloxamer 188 is dissolved in water, followed by dispersion of simethicone and drug substance. Separately, methyl and propylparabens are dissolved in propylene glycol to form a preservative solution. Citric acid, sodium citrate, and sodium saccharin are separately dissolved in water. Avicel® RC591 is then dispersed into the poloxamer 188/simethicone/drug substance mixture and homogenized. The preservative solution is then admixed and homogenized, followed by the sorbitol solution, buffer solution, and flavor. Alternately, the poloxamer 188 is dissolved in water, followed by dispersion of drug substance. Separately, methyl and propylparabens are dissolved in propylene glycol to form a preservative solution. Citric acid, sodium citrate, simethicone and sodium saccharin are separately dissolved/dispersed in water. Avicel© RC591 is then dispersed into the sorbitol solution and homogenized. The preservative solution is then admixed, followed by the sorbitol solution, buffer solution, and flavor. The poloxamer 188/drug susbstance dispersion is then admixed to form the final suspension.

Other formulations can be prepared as indicated above, substituting the other surfactants for poloxamer 188, with the following ingredients:

**Table 2**

| Ingredient | Amount (mg/ml) |
|---|---|
| 5-methyl-2-(2'-chloro-6'-fluoroanilino) phenylacetic acid | 15.0 |
| Propylparaben | 0.20 |
| Poloxamer 188 | 2.00 |
| Methylparaben | 1.80 |
| Simethicone emulsion 30% | 1.00 |
| Flavor | 5.0 |
| Suspending agent: Avicel® RC591 | 15.00 |
| Propylene glycol | 25.00 |
| Sorbitol solution 70% | 250.00 |
| Citric acid anhydrous | 0.71 |
| Sodium citrate dihydrate | 1.88 |
| Sodium saccharin | 0.50 |
| Water purified, USP | q.s. to 1 ml |

**Table 3**

| Ingredient | Amount mg/ml |
|---|---|
| 5-methyl-2-(2'-chloro-6'-fluoroanilino) phenylacetic acid | 12.5 |
| Propylparaben | 0.20 |
| Polysorbate 80 | 2.00 |
| Methylparaben | 1.80 |
| Simethicone emulsion 30% | 1.00 |
| Flavor | 5.0 |
| Suspending agent: Avicel® RC591 | 15.00 |
| Propylene glycol | 25.00 |
| Sorbitol solution 70% | 250.00 |
| Citric acid anhydrous | 0.71 |
| Sodium citrate dehydrate | 1.88 |
| Ascorbic acid | 10 |
| Sodium saccharin | 0.50 |
| Water purified, USP | q.s. to 1 ml |

**Table 4**

| Ingredient | Amount mg/ml |
|---|---|
| 5-methyl-2-(2'-chloro-6'-fluoroanilino) phenylacetic acid | 15.0 |
| Propylparaben | 0.20 |
| Methylparaben | 1.80 |
| Simethicone emulsion 30% | 1.00 |
| Flavor | 5.0 |
| Suspending agent: Avicel® RC591 | 18.00 |
| Propylene glycol | 25.00 |
| Sorbitol solution 70% | 250.00 |
| Citric acid anhydrous | 3.47 |
| Sodium citrate dehydrate | 9.37 |
| Hydroxyethylcellulose | 1.25 |
| Poloxamer 188 | 2.0 |
| Water purified, USP | q.s. to 1 ml |

**Table 5**

| Ingredient | Amount mg/ml |
|---|---|
| 5-methyl-2-(2'-chloro-6'-fluoroanilino) phenylacetic acid | 15.0 |
| Propylparaben | 0.20 |
| Methylparaben | 1.80 |
| Simethicone emulsion 30% | 1.00 |
| Flavor | 5.0 |
| Suspending agent: Avicel® RC591 | 12.00 |
| Propylene glycol | 25.00 |
| Sorbitol solution 70% | 250.00 |
| Citric acid anhydrous | 3.47 |
| Sodium citrate dehydrate | 9.37 |
| Sodium carboxymethylcellulose | 1.25 |
| Poloxamer 188 | 2.0 |
| Water purified, USP | q.s. to 1 ml |

**Table 6**

| Ingredient | Amount (mg/ml) |
|---|---|
| 5-methyl-2-(2'-chloro-6'-fluoroanilino) phenylacetic acid | 15.0 |
| Propylparaben | 0.16 |
| Poloxamer 188 | 2.00 |
| Methylparaben | 1.48 |
| Simethicone emulsion 30% | 2.00 |
| Flavor | 4.0 |
| Suspending agent: Avicel® RC591 | 15.00 |
| Propylene glycol | 25.00 |
| Sorbitol solution 70% | 250.00 |
| Citric acid anhydrous | 0.71 |
| Sodium citrate dihydrate | 1.88 |
| Sodium saccharin | 0.50 |
| Water purified, USP | q.s. to 1 ml |

**Table 7**

| Ingredient | Amount (mg/ml) |
|---|---|
| 5-methyl-2-(2'-chloro-6'-fluoroanilino) phenylacetic acid | 15.0 |
| Propylparaben | 0.16 |
| Poloxamer 188 | 2.00 |
| Methylparaben | 1.48 |
| Simethicone emulsion 30% | 1.00 |
| Flavor | 4.0 |
| Suspending agent: Avicel® RC591 | 15.00 |
| Propylene glycol | 25.00 |
| Sorbitol solution 70% | 250.00 |
| Citric acid anhydrous | 0.71 |
| Sodium citrate dihydrate | 1.88 |
| Sodium saccharin | 0.50 |
| Water purified, USP | q.s. to 1 ml |

## Claims

1. A liquid oral dosage formulation comprising water, 5-methyl-2-(2'-chloro-6'-fluoroanilino)phenylacetic acid, and a suspending agent, wherein the pH of said formulation is between 4.3 and 5.5.

2. The liquid oral dosage formulation of claim 1, wherein said suspending agent is a member selected from the group consisting of microcrystalline cellulose, carboxymethylcellulose sodium, guar gum, xanthan gum, gellan gum, carrageenan, sodium starch glycolate, and mixtures thereof.

3. The liquid oral dosage formulation of claim 2, further comprising a wetting agent.

4. The liquid oral dosage formulation of claim 3, wherein said wetting agent is a member selected from the group consisting of polysorbate 80, poloxamers, polyethoxylated castor oil, polyethoxylated hydrogenated castor oil, polyoxyl 40 stearate, and mixtures thereof.

5. The liquid oral dosage formulation of claim 2, wherein the pH of said formulation is between 4.5 and 5.5.

6. The liquid oral formulation of claim 6, wherein the pH of said formulation is between 4.75 and 5.25.

7. The liquid oral formulation of claim 7, wherein the pH of said formulation is 5.0, and said poloxamer is poloxamer 188.

8. The liquid oral formulation of claim 1, wherein said suspending agent is a mixture of microcrystalline cellulose and carboxymethylcellulose sodium.

9. The liquid oral formulation of claim 8 comprising a buffer system.

10. The liquid oral formulation of claim 9 wherein said buffer system comprises a member selected from the group consisting of alkaline metal citrate salts with citric acid, alkaline metal acetate salts with acetic acid, alkaline metal succinate salts with succinic acid, and mixtures thereof.

11. The liquid oral formulation of claim 9, further comprising an antifoaming agent.

12. The liquid oral formulation of claim 9, further comprising a preservative.

13. The liquid oral formulation of claim 12, wherein said preservative is a member selected from the group consisting of benzoic acid, sorbic acid, butylparaben, ethylparaben, methylparaben, propylparaben, sodium benzoate, sodium propionate, and mixtures thereof.

14. A method for preparing a liquid oral suspension comprising 5-methyl-2-(2'-chloro-6'-fluoroanilino)phenylacetic acid, comprising:
admixing water, drug substance, and suspending agent, to yield a first mixture, and then admixing buffer system components; or
admixing water, suspending agent and buffer system components to yield a first mixture, and then admixing drug substance.

15. The method of claim 14, wherein said suspending agent is a mixture of microcrystalline cellulose and carboxymethylcellulose sodium.

16. The method of claim 15, wherein said liquid oral suspension has a pH of between 4.3 and 5.5.

17. The method of claim 16, wherein said buffer system components are citric acid and sodium citrate.

18. The method of claim 17, wherein said liquid oral suspension has a pH of 5.0.

19. A method for minimizing the dissolution and degradation of an aqueous suspension of 5-methyl-2-(2'-chloro-6'-fluoroanilino)phenylacetic acid, comprising providing an aqueous suspension of 5-methyl-2-(2'-chloro-6'-fluoroanilino)phenylacetic acid and adjusting the pH of said suspension to between 4.3 and 5.5.

20. The method of claim 19, wherein said pH is adjusted to 5.0.

21. Use of the liquid oral dosage formulation of claim 1 for the manufacture of a medicament for treating a cyclooxygenase-2 dependent disorder or condition.

## Patentansprüche

1. Flüssige orale Dosierungsformulierung, umfassend Wasser, 5-Methyl-2-(2'-chlor-6'-fluoranilino)-phenylessigsäure und ein Suspendiermittel, worin der pH Wert dieser Formulierung zwischen 4,3 und 5,5 liegt.

2. Flüssige orale Dosierungsformulierung nach Anspruch 1, worin das Suspendiermittel ausgewählt ist aus der Gruppe, die besteht aus mikrokrstalliner Cellulose, Carboxymethylcellulosenatrium, Guargummi, Xanthangummi, Gellangummi, Carrageenan, Natriumstärkeglycolat und Gemischen hiervon.

3. Flüssige orale Dosierungsformulierung nach Anspruch 2, die ferner ein Netzmittel enthält.

4. Flüssige orale Dosierungsformulierung nach Anspruch 3, worin das Netzmittel ausgewählt ist aus der Gruppe, die besteht aus Polysorbat 80, Poloxameren, polyethoxyliertem Rizinusöl, polyethoxyliertem hydriertem Rizinusöl, Polyoxyl-40-stearat und Gemischen hiervon.

5. Flüssige orale Dosierungsformulierung nach Anspruch 2, worin der pH Wert dieser Formulierung zwischen 4,5 und 5,5 liegt.

6. Flüssige orale Formulierung nach Anspruch 6, worin der pH Wert dieser Formulierung zwischen 4,75 und 5,25 liegt.

7. Flüssige orale Formulierung nach Anspruch 7, worin der pH Wert dieser Formulierung bei 5,0 liegt und das Poloxamer Poloxamer 188 ist.

8. Flüssige orale Formulierung nach Anspruch 1, worin das Suspendiermittel ein Gemisch von mikrokristalliner Cellulose und Carboxymethylcellulosenatrium ist.

9. Flüssige orale Formulierung nach Anspruch 8, die ein Puffersystem enthält.

10. Flüssige orale Formulierung nach Anspruch 9, worin das Puffersystem ausgewählt ist aus der Gruppe, die besteht aus Alkalimetallcitratsalzen mit Citronensäure, Alkalimetallacetatsalzen mit Essigsäure, Alkalimetallsuccinatsalzen mit Bernsteinsäure und Gemischen hiervon.

11. Flüssige orale Formulierung nach Anspruch 9, die ferner ein Antischaummittel enthält.

12. Flüssige orale Formulierung nach Anspruch 9, die ferner ein Konservierungsmittel enthält.

13. Flüssige orale Formulierung nach Anspruch 12, worin das Konservierungsmittel aus der Gruppe ausgewählt ist, die besteht aus Benzoesäure, Sorbinsäure, Butylparaben, Ethylparaben, Methylparaben, Propylparaben, Natriumbenzoat, Natriumpropionat und Gemischen hiervon.

14. Verfahren zur Verstellung einer flüssigen oralen Suspension, die 5-Methyl-2-(2'-chlor-6'-fluoranilino)-phenylessigsäure enthält, umfassend
Vermischung von Wasser, Wirkstoff und Suspendiermittel unter Erhalt eines ersten Gemisches und anschließende Zumischung der Puffersystemkomponenten, oder
Vermischung von Wasser, Suspendiermittel und Puffersystemkomponenten unter Erhalt eines ersten Gemisches und anschließend Zumischung des Wirkstoffs.

15. Verfahren nach Anspruch 14, worin das Suspendiermittel ein Gemisch von mikrokristalliner Cellulose und Carboxymethylcellulosenatrium ist.

16. Verfahren nach Anspruch 15, worin die flüssige orale Suspension einen pH Wert zwischen 4,3 und 5,5 hat.

17. Verfahren nach Anspruch 16, worin die Puffersystemkomponenten Citronensäure und Natriumcitrat sind.

18. Verfahren nach Anspruch 17, worin die flüssige orale Suspension einen pH Wert von 5,0 hat.

19. Verfahren zur Minimierung der Auflösung und des Abbaus einer wässrigen Suspension von 5-Methyl-2-(2'-chlor-6'-fluoranilino)-phenylessigsäure, durch Bereitstellung einer wässrigen Suspension von 5-Methyl-2-(2'-chlor-6'-fluoranilino)-phenylessigsäure und Einstellung des pH Werts der Suspension zwischen 4,3 und 5,5.

20. Verfahren nach Anspruch 19, worin der pH Wert auf 5,0 eingestellt wird.

21. Verwendung der flüssigen oralen Dosierungsform nach Anspruch 1 zur Herstellung eines Arzneimittels für die Behandlung einer von Cyclooxygenase-2 abhängigen Störung oder eines solchen Zustands.

## Revendications

1. Formulation posologique orale liquide comprenant de l'eau, de l'acide 5-méthyl-2-(2'-chloro-6'-fluoroanilino)phénylacétique et un agent de suspension, où le pH de ladite formulation est compris entre 4,3 et 5,5.

2. Formulation posologique orale liquide selon la revendication 1, dans laquelle ledit agent de suspension est un membre choisi dans le groupe constitué par la celullose microcristalline, la carboxyméthylcellulose sodique, la gomme guar, la gomme de xanthane, la gomme gellane, la carragénine, le glycolate d'amidon sodique, et leurs mélanges.

3. Formulation posologique orale liquide selon la revendication 2, comprenant en outre un agent mouillant.

4. Formulation posologique orale liquide selon la revendication 3, dans laquelle ledit agent mouillant est un membre choisi dans le groupe constitué par le polysorbate 80, les poloxamers, l'huile de ricin polyéthoxylée, l'huile de ricin hydrogénée polyéthoxylée, le stéarate de polyoxyle 40, et leurs mélanges.

5. Formulation posologique orale liquide selon la revendication 2, où le pH de ladite formulation est compris entre 4,5 et 5,5.

6. Formulation orale liquide selon la revendication 6, où le pH de ladite formulation est compris entre 4,75 et 5,25.

7. Formulation orale liquide selon la revendication 7, où le pH de ladite formulation est de 5,0, et ledit poloxamer est le poloxamer 188.

8. Formulation orale liquide selon la revendication 1, dans laquelle ledit agent de suspension est un mélange de cellulose microcristalline et de carboxyméthylcellulose sodique.

9. Formulation orale liquide selon la revendication 8, comprenant un système tampon.

10. Formulation orale liquide selon la revendication 9, dans laquelle ledit système tampon comprend un membre choisi dans le groupe constitué par les sels de citrate de métal alcalin avec l'acide citrique, les sels d'acétate de métal alcalin avec l'acide acétique, les sels de succinate de métal alcalin avec l'acide succinique, et leurs mélanges.

11. Formulation orale liquide selon la revendication 9, comprenant en outre un agent antimoussant.

12. Formulation orale liquide selon la revendication 9, comprenant en outre un conservateur.

13. Formulation orale liquide selon la revendication 12, dans laquelle ledit conservateur est un membre choisi dans le groupe constitué par l'acide benzoïque, l'acide sorbique, le butylparaben, l'éthylparaben, le méthylparaben, le propylparaben, le benzoate de sodium, le propionate de sodium, et leurs mélanges.

14. Procédé de préparation d'une suspension orale liquide, comprenant l'acide 5-méthyl-2-(2'-chloro-6'-fluoroanilino)phénylacétique, comprenant:
le mélange de l'eau, de la substance médicamenteuse et de l'agent de suspension, pour donner un premier mélange, et ensuite l'incorporation des composants du système tampon; ou
le mélange de l'eau, de l'agent de suspension et des composants du système tampon pour donner un premier mélange, et ensuite l'incorporation de la substance médicamenteuse.

15. Procédé selon la revendication 14, dans lequel ledit agent de suspension est un mélange de cellulose microcristalline et de carboxyméthylcellulose sodique.

16. Procédé selon la revendication 15, dans lequel ladite suspension orale liquide a un pH compris entre 4,3 et 5,5.

17. Procédé selon la revendication 16, dans lequel lesdits composants du système tampon sont l'acide citrique et le citrate de sodium.

18. Procédé selon la revendication 17, dans lequel ladite suspension orale liquide a un pH de 5,0.

19. Procédé pour réduire au minimum la dissolution et la dégradation d'une suspension aqueuse d'acide 5-méthyl-2-(2'-chloro-6'-fluoroanilino)phénylacétique, comprenant l'obtention d'une suspension aqueuse d'acide 5-méthyl-2-(2'-chloro-6'-fluoroanilino)phénylacétique et l'ajustement du pH de ladite suspension entre 4,3 et 5,5.

20. Procédé selon la revendication 19, dans lequel ledit pH est ajusté à 5,0.

21. Utilisation de la formulation posologique orale liquide selon la revendication 1 pour la fabrication d'un médicament destiné au traitement d'un trouble ou d'une affection dépendant(e) de la cyclooxygénase-2.
